# EUROPEAN PATENT SPECIFICATION

(11) **EP 0 702 964 B1**
(45) Date of publication and mention of the grant of the patent: **02.01.2002**
(21) Application number: 95300271.4
(22) Date of filing: 17.01.1995
(51) Int. Cl.: A61L 9/03, F17C 7/04

(54) **Gas dispenser**
Gasverteiler
Distributeur de gaz

(30) Priority: 17.09.1994 GB 9418797
(43) Date of publication of application: 27.03.1996
(73) Proprietor: The BOC Group plc, Windlesham Surrey GU20 6HJ (GB)
(72) Inventor: Shervington, Evelyn Arthur, Nr. Petersfield, Hampshire, GH31 5LD (GB); Burningham, Raymond Cyril, Morden, Surrey, SM4 5JW (GB)
(74) Representative: Gough, Peter

(56) References cited:
- FR-A- 1 252 481
- PATENT ABSTRACTS OF JAPAN vol. 16, no. 21 (M-1201) 20 January 1992 & JP-A-03 236 526 (KAJIMA CORP. ET AL.) 22 October 1991

## Description

The present invention relates to apparatus for dispensing a smell/fragrance at a pre-selected location.

It is known to spread a fragrance or fragrances by manually spraying liquid odorous substances in an atomised form. However, this method of spreading a fragrance is not susceptible to a precise method of control.

It is also known to incorporate a fragrance into a solid matrix such as soap or plastic pellets. The rate of dispersal in this instance is unreliable in that it will depend on ambient temperature, humidity and how much fragrance was incorporated initially and how much is left at any time.

A further known method is incorporating the aroma in an oil solvent and heating the oil. This works well initially but the smell alters over time due to oxidation and preferential boil-off of the more volatile parts of the active ingredients.

It is also known to use a fragrance dissolved in alcohol or water and dispersed by forced evaporation with a fan of wicks. Again, this method suffers from the disadvantage of lack of control of the dispersal, oxidation and the rate of dosage.

All the currently available ways of putting smells into an environment are generally unreliable in that obtaining the precise amount of the smell in the pre-selected place required in the form that it is to be used is difficult to do accurately and consistently.

Problems with existing systems revolve around the fact that smells are highly complex and volatile, they can oxidise easily and they change and age. A further factor of unreliability is that the various components of a fragrance and there are often ten or more different components of a fragrance, have different boiling points and evaporate at different rates and this will cause the smell to alter over time.

A further problem with dispersing fragrances is that they are detectable in parts per billion but the normal dispersing modes are relatively inexact so that sprays are difficult to disperse evenly over wide areas.

US Patent 3 001 374 discloses a method of regulating the pressure of carbon dioxide in which the carbon dioxide in liquefied state is initially contained within a high pressure cylinder. The cylinder has a discharge valve which communicates via tubing with a regulating means. The downstream side of the regulating means is connected to a second pressure regulating valve via a conduit to which is connected a plurality of heat conducting fins.

Japanese Patent Publication No. JP 3 236 526, describes an apparatus for dispensing an aroma gas which includes a fluid odour bomb in which an ingredient or ingredients for creating the aroma are contained and stored under pressure in a gas cylinder. An aroma line extends from the gas cylinder and located in said line is a valve for controlling the flow of aroma gas from the gas cylinder.

It is an aim of the present invention to provide an apparatus for dispensing a variety of fragrances and aromas precisely, controllably and reliably in most types of enclosed space.

According to the present invention an apparatus for dispensing a smell/fragrance in the form of an aroma gas at a pre-selected location, comprises an ingredient for creating the smell or fragrance is contained and stored under pressure in a gas cylinder, a first aroma gas line extends from the gas cylinder and a valve located in the first aroma gas line controls the flow of aroma gas from the cylinder, and is characterised in that the ingredient or ingredients for creating the smell/fragrance is/are desolved in a gaseous liquid solvent stored under pressure in the gas cylinder and in that the first aroma gas line includes a restrictive throttle in the form of a pre-determined length of tubing, said valve being located in the first aroma gas line downstream of the tubing, and means downstream of the valve for imparting heat to the aroma gas and a second aroma gas line extending from said means towards a dispersion nozzle.

Preferably said means for applying heat to the aroma gas includes a length of copper tubing.

In a preferred embodiment the valve is a solenoid valve which is operated by timing means

An embodiment of the invention will now be described by way of example reference being made to the accompanying diagrammatic drawing which is a schematic view of an apparatus for dispensing a smell/fragrance in the form of an aroma gas at a pre-selected location.

As shown, an apparatus 1 for dispensing a smell/fragrance at a pre-selected location, for example, a cinema includes a high pressure gas cylinder 2 of approximately 50bar containing an active ingredient or ingredients, that is, a substance which on release from the cylinder 2 will create the desired smell/fragrance, dissolved in a liquid gas solvent. The liquid gas solvent maybe, for example, liquid carbon dioxide and alcohol or liquid nitrous oxide and is herein referred to as "aroma gas". Extending substantially through the length of the cylinder 2 is a dip tube 4 to which is connected a first aroma gas line 5 which includes a restrictive throttle in the form of a predetermined length of very small bore tubing 6 in the order of nineteen thousandth of an inch core diameter (0.483mm).

Located downstream of the tubing 6 is a solenoid valve 8 which can be operated by a timer or computer, not shown, in a manner known per se.

Downstream of the valve 8 is means for applying or imparting heat to the aroma gas which means includes a length of copper tubing 12.

The copper tubing 12 effectively separates the first aroma gas line 5 from a second aroma gas line 14 of small bore tubing having a similar bore size as the line 5 which line 14 acts as a second final throttle part downstream of the tubing 12.

Attached to the distal end of the second line 14 is a dispersion nozzle 10.

In use the solenoid valve 8 will be set to allow aroma gas to exit the cylinder 2 through the dip tube 4 and first aroma gas line 5 including the tubing 6 at a controlled frequency and duration. The tubing 6 acts as a restrictive throttle to ensure that the aroma gas passes slowly therethrough and to provide a small cross-sectional area of gas at the inlet to the solenoid valve. The valve 8 is operated by a timer to open the valve 8 at a given frequency and duration thereby ensuring precise control of each bolus of aroma gas passing through the valve 8. The gas after passing through the valve 8 will when passing through the copper tubing 12, be heated sufficiently to ensure that the aroma gas will boil-off effectively and not freeze up.

The second aroma gas line 14 leads to the dispersing nozzle 10 where the aroma gas is released into the space around the pre-selected location. The second line 14 together with the dispersion nozzle 10 act as a further throttle to ensure a spread of each bolus of aroma gas into the pre-selected location and to minimise noise by slowing the flow of aroma gas down.

It will be appreciated that the dispersing apparatus 1 will depend for its precision and controllability on the relationship of the solubility of the active ingredients in the liquid gas solvent, the viscosity of the resulting mixtures and the ability to hold the active ingredient in the dissolved state in the liquid gas until it is dispersed through the nozzle 10.

The necessity to have the two restrictive throttle parts of precise dimensions and the means for applying heat to the aroma gas will vary depending on the viscosity of the various aromas being handled and is of the greatest importance. The bore diameters in particular will vary. Too small a bore diameter can easily be blocked but too large a bore diameter will allow too much gas through and thus reduce the control of the aroma gas bolus size.

Particular advantages of the apparatus 1 are as follows
a) The aromas are always stored in an inert compound, ie liquid CO₂, and these do not oxidise or otherwise change until dispersed so are always reproducible.
b) As the aroma gas is easily handled the amount dispersed can be consistently known and controlled.
c) The fragrance balance remains the same from beginning to the end of the cylinder contents as no part of the fragrance boils off.
d) The apparatus is virtually noiseless as the gas is throttled through two spaced lengths of hypodermic tubing.
e) The gas solvent ensures that dispersion blockage is minimised.

The apparatus can be plumbed into air conditioning systems for places such as cinemas, shopping arcade or indeed shops. It can also be free standing as long as there is some sort of electrical power supply.

It could also be used to supply odours to systems such as virtual reality computer systems whereby the system described can be incorporated into the operating module to provide smells at appropriate times.

It could also be used in conjunction with a food packaging line to introduce aromas into packaged products, for example potato crisp packets.

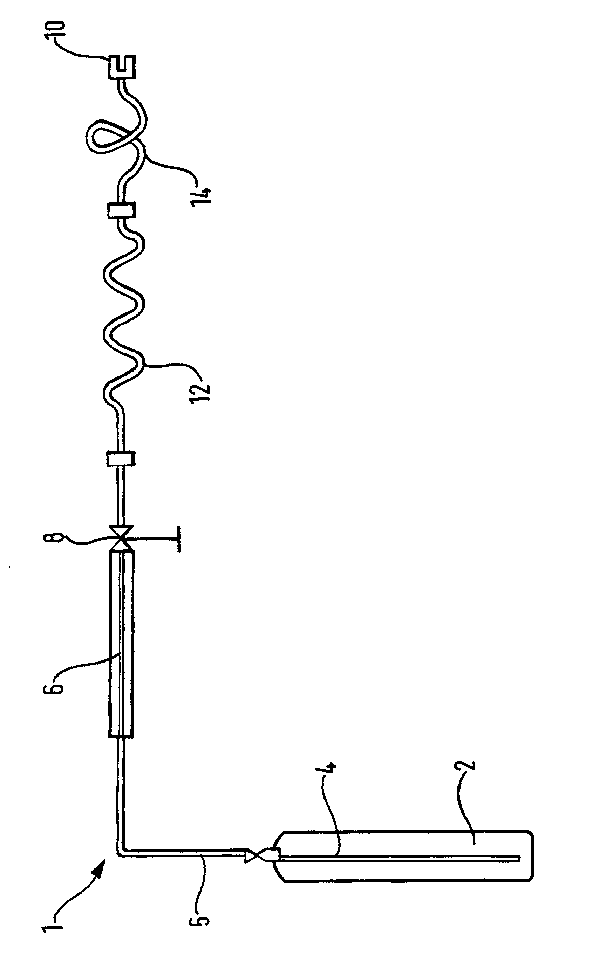

## Claims

1. An apparatus (1) for dispensing a smell/fragrance in the form of an aroma gas at a pre-selected location, comprising an ingredient or ingredients for creating the smell or fragrance contained and stored under pressure in a gas cylinder (2), a first aroma gas line (5) extending from the gas cylinder (2) and a valve (8) located in the first aroma gas line (5) for controlling the flow of aroma gas from the cylinder (2), **characterised in that** the ingredient or ingredients for creating the smell/fragrance is/are desolved in a gaseous liquid solvent stored under pressure in the gas cylinder (2) and **in that** the first aroma gas line (5) includes a restrictive throttle in the form of a pre-determined length of tubing (6), said valve (8) being located in the first aroma gas line downstream of the tubing (6), and means (12) downstream of the valve (8) for imparting heat to the aroma gas and a second aroma gas line (14) extending from said means (12) towards a dispersion nozzle (10).

2. An apparatus as claimed in Claim 1, in which the means includes a length of copper tubing (12).

3. An apparatus as claimed in Claim 1 or 2, in which the valve (8) is a solenoid valve.

4. An apparatus as claimed in Claim 3, in which the solenoid valve (8) is operated by timing means.

## Patentansprüche

1. Einrichtung (1) zur Abgabe eines Geruchs/Dufts in Form eines Aromagases an einem vorgewählten Ort, wobei ein Bestandteil oder Bestandteile zum Erzeugen des Geruchs oder Dufts unter Druck in einem Gaszylinder (2) enthalten und gespeichert ist bzw. sind, eine erste Aromagasleitung (5) vom Gaszylinder (2) ausgeht und ein Ventil (8) in der ersten Aromagasleitung (5) angeordnet ist, um die Strömung von Aromagas aus dem Zylinder (2) zu steuern, **dadurch gekennzeichnet, dass** der Bestandteil bzw. die Bestandteile zum Erzeugen des Geruchs/Dufts in einem gasförmigen flüssigen Lösungsmittel aufgelöst ist bzw. sind, das unter Druck im Gaszylinder (2) gespeichert ist, und das die ersten Aromagasleitung (5) eine restriktive Drossel in Form einer vorgegebenen Länge eines Rohrabschnitts (6) enthält, das Ventil (8) in der ersten Aromagasleitung stromab des Rohrabschnitts (6) angeordnet ist, und Mittel (12) stromab des Ventils (8) zur Mitteilung von Wärme an das Aromagas und eine zweite Aromagasleitung (14) vorgesehen sind, die von den genannten Mitteln (12) ausgehen und zu einer Abgabedüse (10) verlaufen.

2. Einrichtung nach Anspruch 1, wobei die Mittel einen Kupferrohrabschnitt (12) umfassen.

3. Einrichtung nach Anspruch 1 oder 2, wobei das Ventil (8) ein Elektromagnetventil ist.

4. Einrichtung nach Anspruch 3, wobei das Elektromagnetventil (8) durch einen Zeitgeber betätigt wird.

## Revendications

1. Dispositif (1) pour diffuser une odeur/un parfum sous la forme d'un gaz d'arôme en un emplacement présélectionné, comprenant un ingrédient ou des ingrédients destiné(s) à créer l'odeur ou le parfum contenu(s) et stocké(s) sous pression dans un cylindre (2) à gaz, une première canalisation (5) pour le gaz d'arôme, partant du cylindre (2) à gaz, et une valve (8) située dans la première canalisation (5) pour le gaz d'arôme, destinée à contrôler le flux de gaz d'arôme en provenance du cylindre (2), ***caractérisé en ce que*** l'ingrédient ou les ingrédients destiné(s) à créer l'odeur/ le parfum est/sont dissous dans un solvant liquide gazeux stocké sous pression dans le cylindre (2) à gaz et ***en ce que*** la première canalisation (5) de gaz d'arôme comprend un étrangleur restrictif sous la forme d'une longueur prédéterminée de tubulure (6), ladite valve (8) étant située dans la première canalisation pour le gaz d'arôme en aval de la tubulure (6), et des moyens (12) en aval de la valve (8) destinés à communiquer de la chaleur au gaz d'arôme et une seconde canalisation (14) pour le gaz d'arôme partant desdits moyens (12) vers un embout disperseur (10).

2. Dispositif selon la Revendication 1, dans lequel les moyens comprennent une longueur de tubulure de cuivre (12).

3. Dispositif selon la Revendication 1 ou 2, dans lequel la valve (8) est une valve à électro-aimant.

4. Dispositif selon la Revendication 3, dans lequel la valve (8) à électro-aimant est actionnée par des moyens de synchronisation.
